# EUROPÄISCHE PATENTSCHRIFT

(11) **EP 2 134 392 B1**
(45) Veröffentlichungstag und Bekanntmachung des Hinweises auf die Patenterteilung: **06.07.2016**
(21) Anmeldenummer: 08716685.6
(22) Anmeldetag: 25.03.2008
(51) Int. Cl.: A61M 5/24

(54) **VORRICHTUNG ZUR VERABREICHUNG EINES FLUIDEN WIRKSTOFFES AUS EINER MEHRKAMMERKARPULLE**
METHOD FOR ADMINISTERING A FLUID ACTIVE SUBSTANCE FROM A MULTI-CHAMBER CARPULE
DISPOSITIF D'ADMINISTRATION D'UNE PRINCIPE ACTIF FLUIDE DEPUIS UNE CARPULE MULTICAVITÉ

(30) Priorität: 05.04.2007 DE 102007016811
(43) Veröffentlichungstag der Anmeldung: 23.12.2009
(73) Patentinhaber: Tecpharma Licensing AG, 3401 Burgdorf (CH)
(72) Erfinder: GALBRAITH, Sofia, 4500 Solothurn (CH); MOSER, Ulrich, 3412 Heimiswil (CH); HIRSCHEL, Jürg, 5000 Aarau (CH); THOMPSON, Ian, 3400 Burgdorf (CH)
(86) Internationale Anmeldenummer: PCT/EP2008/002341
(87) Internationale Veröffentlichungsnummer: WO 2008/122360

(56) Entgegenhaltungen:
- EP-A- 1 392 377
- WO-A-01/30425
- WO-A-2004/028598
- WO-A-2006/057604
- DE-A1- 10 036 594

## Beschreibung

Die vorliegende Erfindung betrifft eine Verabreichungsvorrichtung zur Verabreichung eines fluiden Wirkstoffes aus einer Mehrkammerkarpule, insbesondere aus einer Zweikammerkarpule oder Mehrkammerampulle.

Verschiedene Wirkstoffe, wie z.B. Wachstumshormone, können in einer in Flüssigkeit gelösten Form nicht über einen längeren Zeitraum aufbewahrt werden. Dennoch ist es zur erfolgreichen Behandlung von z.B. Wachstumsstörungen erforderlich, derartige Wirkstoffe in flüssiger Form in ein Körpergewebe eines Patienten einzubringen. Üblicherweise werden hierfür Verabreichungsvorrichtungen z.B. in Form von Injektionspens, verwendet. Um derartige Wirkstoffe mit einer geringen Haltbarkeit in einem flüssigen Medium für eine Verabreichung mit Injektionsgeräten bereitstellen zu können, wurden Mehrkammerkarpulen, insbesondere Zweikammerkarpulen, entwickelt, in welchen ein Wirkstoff in getrockneter, bzw. lyophilisierter Form in einer ersten Karpulenkammer und eine Lösungsflüssigkeit getrennt von dem Wirkstoff in einer zweiten Karpulenkammer aufbewahrt wird. Meist sind die beiden Kammern durch bewegliche Stopfen voneinander getrennt. Die erste Kammer wird daher durch einen beispielsweise durch eine Membran verschlossenen Ausgang der Karpule, der Karpulenwand und einem ersten Stopfen gebildet. Die zweite Kammer wird anschliessend an die erste Kammer durch den ersten Stopfen, die Karpulenwand und einen zweiten Stopfen gebildet. Entlang der Längsachse der Karpule und somit entlang der Achse in der die Stopfen verschoben werden können, ist in der Karpulenwand ein Bypass vorgesehen, der als Umlenkung um den ersten Stopfen für die Lösungsflüssigkeit dienen kann. Zum Abmischen des Wirkstoffes mit der Lösungsflüssigkeit wird ein Druck auf den zweiten Stopfen innerhalb der Karpule ausgeübt, der durch die inkompressible Lösungsflüssigkeit auf den ersten Stopfen übertragen wird. Die beiden Stopfen verschieben sich dadurch entlang der Längsachse der Karpule relativ zur Karpulenwand. Sobald der erste Stopfen im Bereich des Karpulenbypasses zu liegen kommt, gelangt die Lösungsflüssigkeit durch den Bypass aus der zweiten Kammer in die erste Kammer mit dem Wirkstoff. Der zweite Karpulenstopfen kann so lange relativ zur Karpulenwand und zum ersten Stopfen verschoben werden, bis er an dem ersten Stopfen aufliegt. Bei einem weiteren Verschieben des zweiten Stopfens wird der erste Stopfen mitverschoben und die Lösungsflüssigkeit mitsamt dem Wirkstoff kann durch die Karpulenöffnung ausgeschüttet werden. Hierfür wird eine Injektionsnadel auf die Karpulenöffnung aufgesetzt, welche eine Fluidverbindung zur ersten Karpulenkammer bilden kann. Beim Abmischen des Wirkstoffes mit der Lösungsflüssigkeit ist darauf zu achten, dass der Wirkstoff keinen zu grossen Lösungsflüssigkeitsströmungen ausgesetzt wird. Eine Schaumbildung soll beim Abmischen möglichst vermieden werden. Der Wirkstoff liegt nun in gelöster Form vor und kann mit herkömmlichen Verabreichungsgeräten in ein Gewebe eines Patienten injiziert werden.

Aus der DE 10 2004 055 298 A1 ist eine Abmischvorrichtung für eine Zweikammerkarpule bekannt, bei der eine Zweikammerkarpule in einer Abmischhülse aufgenommen ist, wobei die Abmischhülse in einer axialen Bewegung auf eine Verabreichungsvorrichtung aufgeschoben wird. Beim Aufschieben der Abmischhülse werden die Stopfen der Zweikammerkarpule innerhalb der Karpule verschoben und es erfolgt eine Abmischung des Wirkstoffes mit der Lösungsflüssigkeit. Aus der EP 0 298 067 B1 ist eine Anordnung bekannt, bei der die Karpule in eine Karpulenhülse eingesetzt wird und die Karpulenhülse in das Gehäuse einer Verabreichungsvorrichtung eingeschraubt wird. Durch die Axialbewegung während des Einschraubens werden die Stopfen innerhalb der Zweikammerkarpule verschoben und der Wirkstoff wird mit der Lösungsflüssigkeit vermischt. Die bekannten Geräte aus dem Stand der Technik ermöglichen ein langsames Abmischen des Wirkstoffes, um eine Schaumbildung oder eine Schädigung des Wirkstoffes zu vermeiden.

Für einen Anwender der Vorrichtungen ist es jedoch schwierig zu erkennen, wann der Abmischvorgang vollständig ausgeführt wurde und somit der Wirkstoff in der gewünschten Konzentration in der Lösungsflüssigkeit vollständig gelöst ist. Vor der Verabreichung eines in flüssiger Form vorliegenden Wirkstoffes mittels einer Verabreichungsvorrichtung in ein Gewebe eines Patienten ist es erforderlich, die Flüssigkeitsleitung zwischen der Karpulenkammer, in welcher die Flüssigkeit aufbewahrt wird, und der Spitze einer Injektionsnadel zu entlüften. Dieser Vorgang wird im Allgemeinen als Priming bezeichnet. Der Primingvorgang muss bei den aus dem Stand der Technik bekannten Verabreichungsvorrichtungen für Zweikammerkarpulen aktiv von einem Anwender durchgeführt werden und kann somit vor einer Verabreichung eines flüssigen Wirkstoffes auch vergessen werden.

Der vorliegenden Erfindung liegt die Aufgabe zugrunde, eine Verabreichungsvoirichtung für einen Wirkstoff aus einer Mehrkammerkarpule zu schaffen, welche den Ablauf bei der Verwendung der Vorrichtung vereinfacht und eine sichere Dosierung und Verabreichung des Wirkstoffes sicherstellt.

Gemäss der Erfindung weist eine Verabreichungsvorrichtung für Mehrkammerkarpulen eine Anzeigenvorrichtung auf, die zumindest eine erste Anzeige zur Markierung einer Stellung der Karpule relativ zu einem Gehäuse der Vorrichtung nach Beendigung eines Abmischvorganges und eine zweite Anzeige zur Markierung einer Stellung der Karpule relativ zum Gehäuse der Vorrichtung nach Beendigung eines Entlüftungsvorganges umfasst. Die Anzeige kann dabei auch durch eine Raststellung gegeben sein. Die Mehrkammerkarpule kann, wie eingangs beschrieben, durch eine Zweikammerkarpule gebildet werden. Es ist jedoch auch möglich, mehr als zwei Kammern innerhalb einer solchen Karpule vorzusehen, um z.B. zwei verschiedene in fester Form vorliegende Wirkstoffe oder auch zwei verschiedene Lösungsflüssigkeiten zur Verfügung zu stellen. Zum Abmischen des Wirkstoffes innerhalb der Karpule kann die Verabreichungsvorrichtung beispielsweise ein Antriebsglied in Form einer Kolbenstange aufweisen, welche dazu dient, beim Einsetzen der Mehrkammerkarpule die Stopfen innerhalb der Karpule gegenüber der Karpulenwand vorzuschieben und vorzugsweise anschliessend an das Abmischen die Wirkstoffflüssigkeit aus der Karpule auszuschütten. Der Abmischvorgang kann dabei durch Aufschieben oder Aufdrehen eines Aufnahmegliedes oder Karpulenhalters für die Mehrkammerkarpule auf oder in ein Gehäuse der Verabreichungsvorrichtung erfolgen. Hierfür kann z.B. ein einfaches Gewinde zwischen Gehäuse und Karpulenhalter vorgesehen sein. Es ist jedoch auch möglich, eine Mischvorrichtung mit einer Mechanik mit einer Übersetzung oder einer Mechanik gemäss DE 10 2004 055 298 A1 vorzusehen. Auch andere aus dem Stand der Technik bekannte Abmischvorrichtung können im Sinne der Erfindung verwendet werden. Vorzugsweise werden die Raststellungen zur Anzeige der einzelnen Positionen bei den einzelnen Schritten der Vorbereitung der Verabreichungsvorrichtung, wie dem Abmischen, Entlüften und Aufdosieren durch eine Rückschiebe- oder Rückdrehsicherung gebildet, durch die es nicht mehr möglich ist, das Aufnahmeglied entgegen seiner Einführrichtung aus dem Gehäuse zu entfernen. Dabei ist es auch möglich, die Rückschiebe- oder drehsicherung lösbar zu gestalten, so dass auf Wunsch ein Lösen der Sicherung möglich ist.

Im Ausgangszustand, bzw. der Anfangsposition des Aufnahmeglieds zum Gehäuse, vor dem Abmischen nimmt die Mehrkammerkarpule, bzw. der Karpulenhalter, eine erste Stellung gegenüber dem Gehäuse, bzw. der Kolbenstange der Verabreichungsvorrichtung, ein. Aus dieser Anfangsposition heraus wird die Karpule in Richtung in das Gehäuse bewegt, bis sie in einer Stellung relativ zum Gehäuse angekommen ist, in der das Abmischen des Wirkstoffes mit der Lösungsflüssigkeit beendet ist. Die erfindungsgemässe Anzeigenvorrichtung weist eine erste Anzeige auf, welche diese Stellung, d.h. die Abmischposition, in welcher das Lösungsmittel vollständig aus der zweiten Karpulenkammer in die erste Karpulenkammer mit dem festen Wirkstoff gelangt ist, markiert. Durch eine weitere Bewegung der Karpule in die proximale Richtung in das Gehäuse, bzw. in Richtung auf die Kolbenstange der Verabreichungsvorrichtung erfolgt ein Entlüftungsvorgang, der in der ersten Kammer verbliebene Luftreste aus der Karpule entfernen soll. Hierfür werden die Stopfen der Karpule gemeinsam verschoben. Diese zweite Stellung, die Entlüflungsposition, der Karpule relativ zum Gehäuse, in welcher der Entlüftungsvorgang beendet ist, wird durch eine zweite Anzeige der Anzeigenvorrichtung markiert.

Mit einer Verabreichungsvorrichtung gemäss der vorliegenden Erfindung kann ein Anwender leicht an der Verabreichungsvorrichtung ablesen oder durch akustische Wahrnehmung erkennen, ob der Abmischvorgang und der anschliessende Primingvorgang vollständig und ordnungsgemäss durchgeführt wurden. Er kann somit sicher sein, dass die Verabreichungsvorrichtung zur Verabreichung des Wirkstoffes bereit ist und bei der Verabreichung eine korrekte Dosis abgegeben wird.

In einer Ausführungsform der Erfindung weist die Anzeigenvorrichtung eine dritte Anzeige auf, welche die Anfangsposition der Karpule relativ zum Gehäuse, bzw. relativ zur Kolbenstange, d.h. der Stellung der Stopfen innerhalb der Karpule, vor dem Beginn eines Abmischvorgangs markiert. Da der Abmischvorgang über eine längere Strecke der Karpule relativ zum Gehäuse, bzw. der Stopfen innerhalb der Karpule, erfolgt, ist es auch möglich, die dritte Anzeige durch mehrere Anzeigeelemente zu gestalten, welche den Fortschritt des Abmischens angeben. Hierfür kann z.B. eine Abfolge von Zahlen oder Zeichen vorgesehen sein oder auch ein sich im Verlauf des Abmischens verjüngenderer oder erweitenderer Balken oder es kann wiederholt ein Rastgeräusch hörbar sein. Die visuellen Anzeigen der Anzeigevorrichtung können entlang einer Längsachse der Verabreichungsvorrichtung axial versetzt angeordnet sein. Die Anzeigen können aber auch in Umfangsrichtung auf dem Umfang der Verabreichungsvorrichtung angeordnet sein. Die Anzeigen können auch in einer Kombination dieser Möglichkeiten vorgesehen sein, d.h. die einzelnen Anzeigen sind sowohl axial als auch in Umfangsrichtung versetzt angeordnet.

In einer Ausführungsform umfasst die Verabreichungsvorrichtung wie oben beschrieben ein Gehäuse und ein Aufnahmeglied in Form eines Karpulenhalters, wobei der Karpulenhalter zum Abmischen des Wirkstoffes und der Lösungsflüssigkeit relativ zum Gehäuse der Verabreichungsvorrichtung beweglich ist. Auf der Karpulenhülse oder auf dem Gehäuse kann ein Zeiger oder eine Aussparung vorgesehen sein, welche eine visuelle Anzeige während der Bewegung des Karpulenhalters relativ zum Gehäuse anwählen, bzw. identifizieren können. Auf dem jeweils anderen der Teile Karpulenhalter und Gehäuse sind die visuelle Anzeigen der Anzeigevorrichtung vorgesehen. Durch die Bewegung des Karpulenhalters relativ zum Gehäuse kann z.B. der Zeiger auf dem Karpulenhalter zuerst auf die Anzeige der Anfangsposition weisen, anschliessend auf die Anzeige der Abmischposition und zuletzt auf die Anzeige der Endstellung des Primings, der Entlüftungsposition. Ebenso ist es möglich, dass die Anzeigen innerhalb einer Aussparung, oder einer Öffnung des Karpulenhalters in der jeweiligen Stellung erscheinen. Letztlich ist auch eine Kombination der Verteilung der einzelnen Anzeigen auf Karpulenhalter und Gehäuse möglich. Es kann z.B. die Anzeige für die Anfangsposition auf dem Karpulenhalter vorgesehen sein und die Anzeige für die Abmischpostion und die Priming- bzw. Entlüftungsposition auf dem Gehäuse vorgesehen sein. Entsprechend sind auf Karpulenhalter und Gehäuse Zeiger vorgesehen, welche die Anzeigen identifizieren.

In dem beschriebenen Ausführungsbeispiel sind die Anzeigen durch visuell wahrnehmbare Markierungen vorgesehen. Als visuelle Anzeigeelemente können, wie bereits angegeben, Ziffern, Zahlen, Farbkodierung, unterschiedliche Formen wie Dreiecke, Vierecke, Kreise und Punkte, verwendet werden. Selbstverständlich ist auch eine Kombination dieser Anzeigeelemente möglich.

Wie bereits beschrieben, kann eine erfindungsgemässe Anzeigenvorrichtung einer Verabreichungsvorrichtung auch an der Karpule selbst vorgesehen sein. In diesem Fall können die Anzeigen durch Markierungen entlang der Karpule angeordnet sein und die Zeiger zum Anwählen der Markierungen können z.B. an dem Stopfen der Karpule, insbesondere dem zweiten Stopfen der Karpule, angeordnet sein. Als Zeiger kann auch eine vordere oder hintere Kante des Stopfens dienen. Ist die Zweikammerkarpule in eine Karpulenhülse eingesetzt, kann an dieser Karpulenhülse beispielsweise ein länglicher Sichtschlitz vorgesehen sein, durch welchen hindurch die Anzeigevorrichtung an der Mehrkammerkarpule abgelesen werden kann.

Nach einem Verfahren gemäss der vorliegenden Erfindung zur Bereitstellung einer Verabreichungsvorrichtung mit einer Mehrkammerkarpule wird die Beendigung des Abmischvorgangs durch eine erste Anzeige angegeben und die Beendigung eines Entlüftungsvorgangs durch eine zweite Anzeige angegeben.

Vorzugsweise gibt eine dritte Anzeige die Ausgangsstellung der Mehrkammerkarpule relativ zu einem Gehäuse der Verabreichungsvorrichtung an. Während des Abmischens der Zweikammerkarpule, d.h. während des Verschiebens der Stopfen innerhalb der Karpule, erscheint bei der Beendigung des Abmischvorgangs zunächst die erste Anzeige. Bei der weiteren Bewegung der Stopfen innerhalb der Karpule erscheint die zweite Anzeige, um das Ende der Entlüftung anzugeben.

Grundsätzlich ist es auch möglich, die Anzeigen durch akustisch wahrnehmbare Signale bereitzustellen. Beispielsweise ist es möglich, die Anzeige der Abmischendstellung durch ein Klicksignal und die Anzeige der Entlüftungsendstellung durch eine sich von dem Klicksignal unterscheidendes Klopfsignal zu signalisieren. Es können aber auch alle Positionen durch das gleiche Geräusch angezeigt werden. Das Geräusch des Klickens oder Klopfens kann beispielsweise durch mechanische Rast- oder Anschlageinrichtungen innerhalb der Verabreichungsvorrichtung vorgesehen werden, welche während dem Abmischen der Zweikammerkarpule betätigt werden.

Bei einer weiteren Ausführungsform der vorliegenden Erfindung ist bei der Vorrichtung zur Verabreichung eines fluiden Produkts eine Rast-, bzw. Anzeigeeinrichtung, für das Einführen des Aufnahmeglieds in das Gehäuse vorgesehen. Um einen Missbrauch des Geräts zu vermeiden, wird zum einen sichergestellt, dass eine einmal begonnene Einfuhr des Aufnahmeglieds in das Gehäuse nach dem Abschluss eines jeden einzelnen Vorbereitungsschrittes nicht rückgängig gemacht werden kann. Zum andern wird der Abschluss der einzelnen Vorbereitungsschritte durch eine Anzeige angezeigt. Dabei kann die Anzeige durch den akustisch wahrnehmbaren Rastton gegeben sein. Hierfür sind beispielsweise Arretierungsmittel vorgesehen, durch welche das Aufnahmeglied nach jedem Vorbereitungsschritt gegen eine Bewegung entgegen der Einfuhrrichtung in das Gehäuse blockiert wird. Dabei ist es wahlweise auch möglich, die Arretierung lösbar auszubilden, sollte dies sinnvoll erscheinen. Im Falle einer Gewindeführung zwischen Gehäuse und Aufnahmeglied bildet die Arretierung beispielsweise eine Rückdrehsicherung gegen ein Wiederherausdrehen des Aufnahmegliedes aus dem Gehäuse. Eine solche Rückdrehsicherung kann beispielsweise durch einen Schnapparm am Aufnahmeglied oder am Gehäuse realisiert werden, der in der Rückdrehrichtung in eine Ausnehmung in dem Gehäuse bzw. dem Aufnahmeglied einrastet, in der Vorwärtsbewegung jedoch aus der Ausnehmung gleiten kann. Eine solche Rückdrehsicherung ist z.B. bei Beendigung des Abmischvorgangs und des Entlüftungsvorgangs vorgesehen. Zudem kann eine solche Rückdrehsicherung bereits beim Einsetzen des Aufnahmeglieds in das Gehäuse vorgesehen werden. D.h. auch in der Ausgangsposition ist das Aufnahmeglied bereits gegen ein vollständiges Herausdrehen aus dem Gehäuse durch eine Arretierung gesichert. Die Verrastung bildet gleichzeitig die Anzeige, dass der entsprechende Vorbereitungsschritt abgeschlossen ist. In der eingeführten Position nach dem Abmischen und Entlüften kann die Arretierung derart ausgebildet sein, dass auch eine Bewegung in proximaler Richtung, d.h. eine Einschraubbewegung, blockiert wird. Demnach ist das Aufnahmeglied in der eingeführten Position nach dem Entlüften sowohl gegen ein Ein- wie auch ein Ausführen, bzw. Ein- oder Ausdrehen, gesichert.

Die Arretierungen, bzw. Rückdrehsicherungen sind demnach derart ausgebildet, dass beim Einrasten oder Einschnappen in die Arretierung zugleich ein akustisches Signal entsteht, so dass der Abschluss der einzelnen Vorbereitungsschritte hörbar wird. Einem Anwender wird daher die Abfolge der einzelnen Vorbereitungsschritte akustisch angezeigt.

Grundsätzlich bildet allein die Rückschiebe- oder drehsicherung für das Aufnahmeglied den Stand der Technik in vorteilhafter Weise weiter, auch ohne dass dabei eine Anzeige erfolgt. Die Anmelderin behält es sich vor, hierauf unabhängige Ansprüche zu richten.

Die Zeichnung zeigt zwei Ausführungsbeispiele einer erfindungsgemässen Verabreichungsvorrichtung. Aus den Figuren hervorgehende Merkmale gelten als von der Erfindung umfasst, wobei die Figuren in keiner Weise einschränkend verstanden werden soll.

In der Zeichnung zeigen:
- Figur 1A:: Verabreichungsvorrichtung gemäss einer ersten Ausführungsform der Erfindung in einer Ausgangsposition,
- Figur 1B:: Detailansicht der Verabreichungsvorrichtung aus Figur 1A in einer um 90° gedrehten Ansicht,
- Figur 2:: Verabreichungsvorrichtung nach der ersten Ausführungsform in einer Abmischposition,
- Figur 3:: Verabreichungsvorrichtung nach der ersten Ausführungsform in einer Entlüftungsposition,
- Figur 4A:: Verabreichungsvorrichtung nach der ersten Ausführungsform in einer eingeführten Position,
- Figur 4B:: Detailansicht analog Figur 1B in der eingeführten Position,
- Figur 5A:: Verabreichungsvorrichtung nach ersten Ausführungsform in einer Endposition,
- Figur 5B:: Detailansicht analog Figur 1B in der Endposition,
- Figur 6A:: Längsschnitt durch die Verabreichungsvorrichtung nach der ersten Ausführungsform mit Rasteinrichtung und
- Figur 6B:: Verabreichungsvorrichtung nach Figur 6A in einer Entlüftungsposition,
- Figur 7: Verabreichungsvorrichtung gemäss der einer zweiten Ausführungsform der Erfindung in einer schematischen Darstellung mit einer visuellen Anzeigenvorrichtung gemäss der vorliegenden Erfindung und
- Figur 8: eine schematische Darstellung von Anzeigeelementen aus Figur 8.

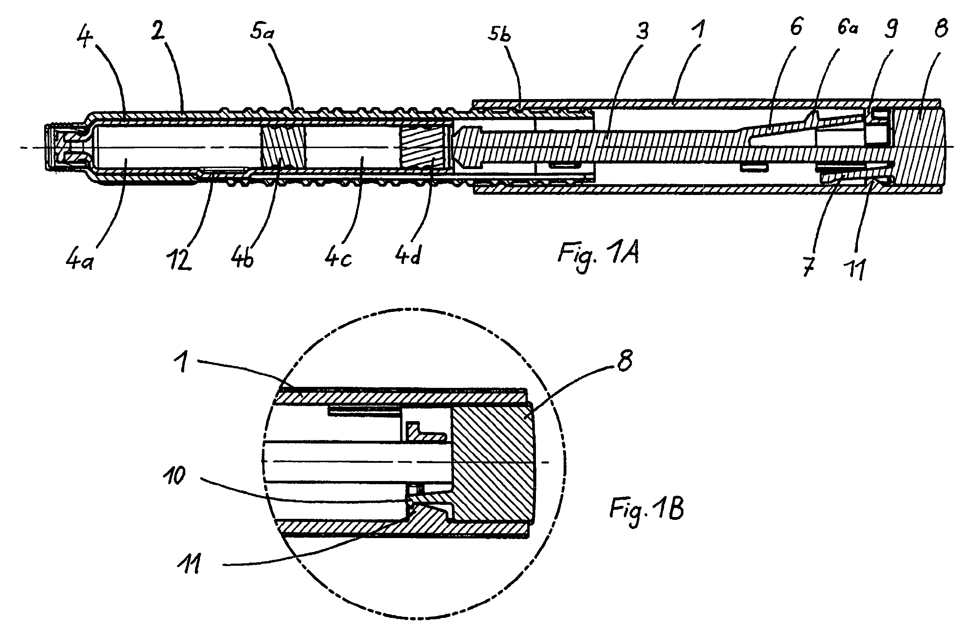

In Figur 1A ist eine erste Ausführungsform einer Vorrichtung zur Verabreichung eines fluiden Produkts gemäss der vorliegenden Erfindung in einer Ausgangsposition gezeigt. Die Vorrichtung weist ein Gehäuse 1, ein Aufnahmeglied 2 und ein Antriebsglied 3 auf. Die Vorrichtung gemäss der Erfindung kommt allein mit diesen drei Bauteilen aus. Lediglich ein Produktreservoir mit einem gewünschten zu verabreichenden Produkt und eine passende Injektionsnadel sind noch zu ergänzen.

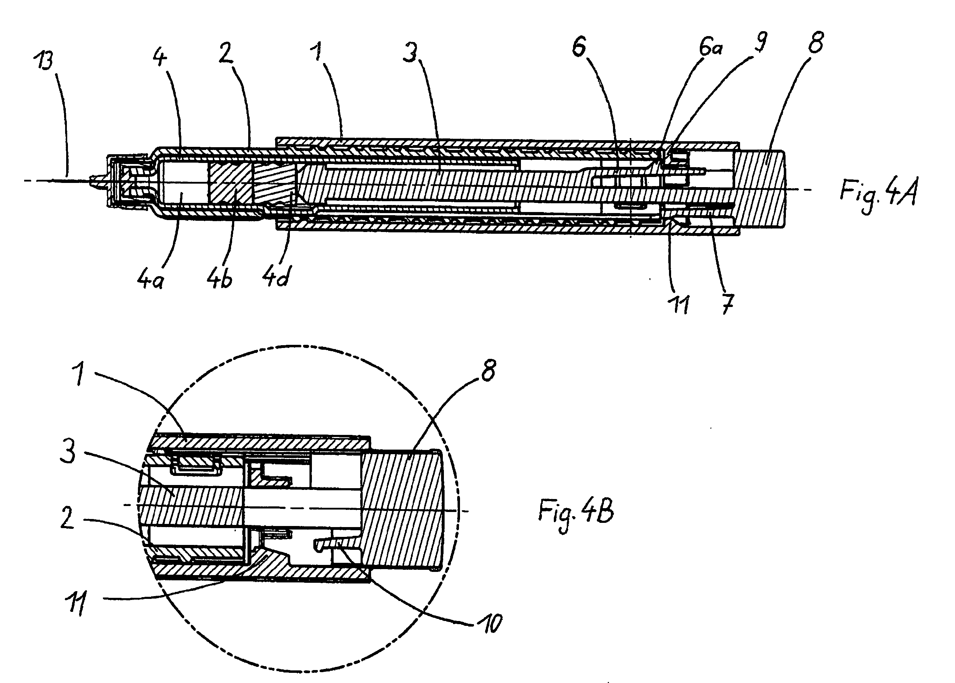

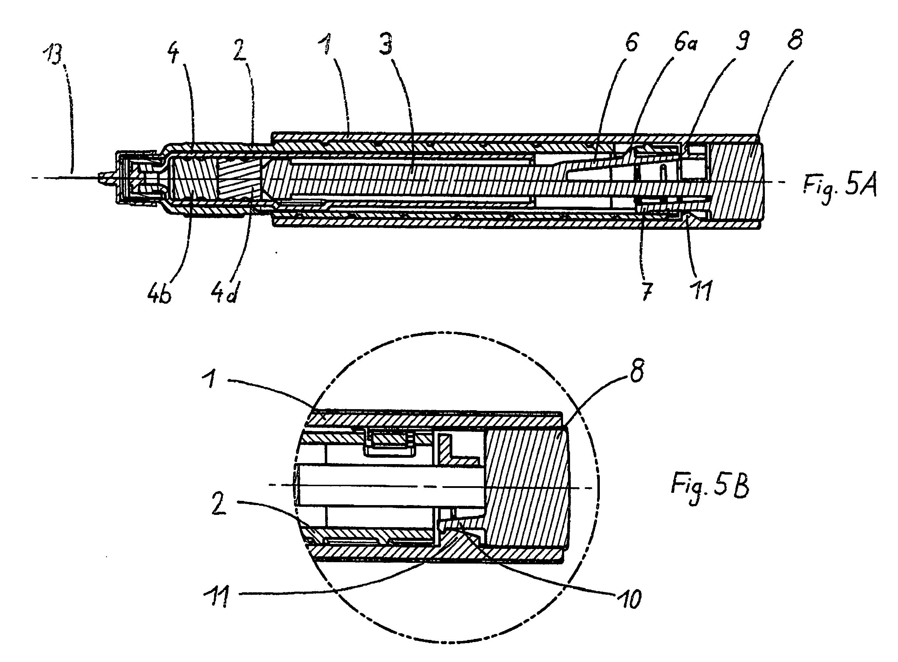

Gemäss dem dargestellten Ausfuhrungsbeispiel ist das Produktreservoir ein Zweikammerreservoir, bzw. eine Zweikammerkarpule, das eine erste Kammer 4a, die durch einen Stopfen 4b begrenzt ist, und eine zweite Kammer 4c umfasst, die durch den ersten Stopfen 4b und einen Stopfen 4d begrenzt ist. Im Umfang der Wand des Produktreservoirs ist zudem ein Bypass 12 (vgl. Figuren 4a und 5a) vorgesehen, durch den wie aus dem Stand der Technik bekannt, Lösungsmittel aus der zweiten Kammer 4c in die Produktkammer 4a umgeleitet werden kann.

Das Aufnahmeglied 2 ist an seinem proximalen Ende teilweise in das distale Ende des Gehäuses 1 eingesetzt. Zwischen dem Gehäuse 1 und dem Aufnahmeglied 2 ist eine Führungsstruktur vorgesehen, welche eine Relativbewegung zwischen Gehäuse 1 und Aufnahmeglied 2 führt. Die Führungsstruktur umfasst eine gewindeartig auf der Aussenumfangsfläche des hülsenförmigen Aufnahmeglieds 2 umlaufende Führungsnut 5a. Auf die Innenumfangsfläche des ebenfalls hülsenförmig ausgebildeten Gehäuses 1 ist ein Führungsnocken 5b vorgesehen, der innerhalb der Führungsnut 5a geführt ist. Der Führungsnocken 5b kann auch als Führungsschiene oder als Führungssegment ausgebildet sein. Die Führungsstruktur 5a, 5b ist in diesem Ausführungsbeispiel als Gewindeführung ausgeführt. Durch eine Rotationsbewegung des Aufnahmeglieds 2 relativ zum Gehäuse 1 wird das Aufnahmeglied 2 in proximaler Richtung axial in das Gehäuse 1 eingeführt.

Das Antriebsglied 3 ist im Wesentlichen stangenförmig ausgebildet. In dem Ausgangszustand gemäss Figur 1A kommt das distale Ende des Antriebsglieds 3 gegenüber dem proximalen Stopfen 4d des Produktreservoirs 4 zu liegen.

Das Verabreichungsgerät in der Ausgangsposition gemäss Figur 1A entspricht einem Auslieferungszustand, in dem sich die Vorrichtung befindet, wenn sie an einen Anwender ausgegeben wird.

Das Antriebsglied 3 besitzt an seinem proximalen Ende ein Halteelement 6 und ein Aktivierungselement 7. Das Halteelement 6 und das Aktivierungselement 7 sind vorzugsweise integraler Bestandteil des Antriebsglieds 3. Es ist jedoch auch möglich, das Halteelement 6 und das Aktivierungselement 7 an den stangenförmigen Bereich des Antriebsglieds anzufügen. Weiter weist das Antriebsglied 3 am proximalen Ende eine Verdickung auf, welche als Antriebsknopf 8 bezeichnet werden kann. Der Antriebsknopf 8 ist in der Ausgangsposition für einen Anwender unzugänglich, da er im Gehäuse 1 versenkt ist.

Das Halteelement 6 und das Aktivierungselement 7 ragen seitlich als flexible Fortsätze oder Arme vom Antriebsglied ab. Grundsätzlich ist es möglich auch zwei oder mehr solcher Arme am Antriebsglied vorzusehen. Das Halteelement 6 ragt in proximaler Richtung und das Aktivierungselement in distaler Richtung vom Antriebsglied 3 ab.

In der Ausgangsposition gemäss Figur 1A kommt das Halteelement 6 gegenüber einem nach innen ragenden Vorsprung, bzw. einer Kante, 9 des Gehäuses in axialer Richtung zu liegen. Das proximale Ende des Halteelements stösst demnach in axialer Richtung gegen den Gehäusevorsprung 9. In dieser Position ist das Halteelement in seiner Grundstellung. Das Halteelement ist jedoch derart flexibel vorspannbar, dass es in radialer Richtung zur Achse des Antriebsglieds 3 hin bewegt werden kann. Das Aktivierungsglied 7 ragt schräg in distale Richtung von dem Antriebsglied 3 ab. Das Aktivierungselement ist in der Ausgangsposition in einem entspannten Zustand, kann jedoch flexibel in radialer Richtung zur Antriebsgliedachse ausgelenkt werden. Sie übernehmen bei der Vorbereitung der Verabreichungsvorrichtung unterschiedliche Funktionen zur Ablaufsteuerung der einzelnen Vorbereitungsschritte. Sie bilden gemeinsam mit dem Antriebsglied die Steuereinrichtung für die Ablaufsteuerung.

Figur 1B zeigt eine Detailansicht des proximalen Bereichs der Verabreichungsvorrichtung gemäss Figur 1A, in der die Vorrichtung um 90° gedreht ist. Es ist ein Sicherungselement 10 erkennbar, welches mit einem Vorsprung 11 an dem Gehäuse 1 zusammenwirkt. Das Sicherungselement 10 und der Vorsprung 11 bilden zusammen eine Klemmverbindung, um das Antriebsglied 3 zusätzlich zu der Blockierung durch den Anschlag zwischen Halteelement 6 und Gehäusevorsprung 9 sowie dem Anschlag zwischen Antriebsknopf 8 und Gehäusevorsprung 9 zu sichern.

Der Anschlag zwischen dem Halteelement 6 und dem Gehäusevorsprung 9 in axialer Richtung bedingt eine Blockierung der Bewegung des Antriebsglieds in proximaler Richtung relativ zum Gehäuse. Der Anschlag zwischen dem Antriebsknopf 8 und dem Gehäusevorsprung 9 bewirkt hingegen eine Blockierung des Antriebsglieds 3 in distaler Richtung relativ zum Gehäuse.

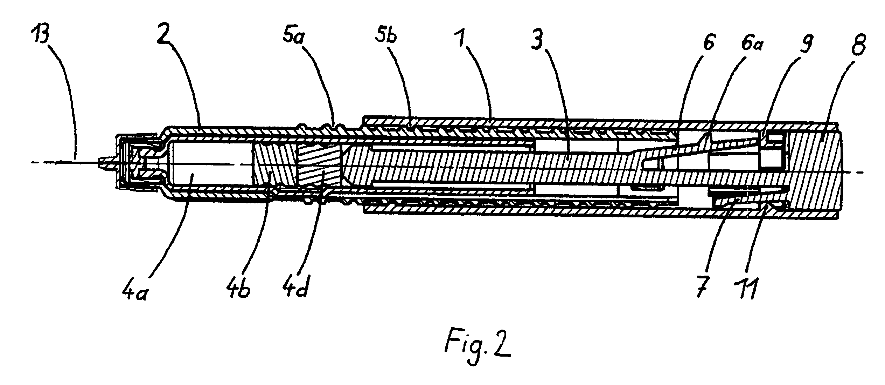

In Figur 2 ist die erfindungsgemässe Verabreichungsvorrichtung in einer Abmischposition gezeigt. Auf das distale Ende der Vorrichtung ist eine Injektionsnadel 13 aufgesetzt. Aus der Ausgangsposition gemäss Figur 1A wird das Aufnahmeglied 2 relativ zum Gehäuse 1 in proximaler Richtung ins Innere des Gehäuses eingeführt bzw. entlang der Führungsstruktur 5a, 5b eingeschraubt. Dabei bleibt das Antriebsglied 3 relativ zum Gehäuse durch die Blockierung zwischen Halteglied 6 und Gehäusevorsprung 9 und auch durch die Sicherung zwischen dem Sicherungselement 10 und dem Vorsprung 11 relativ zum Gehäuse fixiert. Durch das Einführen des Aufnahmeglieds 3 trifft das distale Ende des Antriebsglieds 3 auf den Stopfen 4d des Produktreservoirs 4 und schiebt diesen bei Fortsetzung der Einführbewegung innerhalb des Produktreservoirs in distale Richtung. Dadurch wird zunächst die Antriebskraft durch das Lösungsmittel in der Kammer 4c auf den Stopfen 4b übertragen, so dass beiden Stopfen 4b und 4d distal angetrieben werden. Sobald der Stopfen 4b im Bereich des Bypasses zu liegen kommt, bleibt der Stopfen 4b relativ zum Reservoir 4 in Ruhe. Der Stopfen 4d hingegen wird weiter angetrieben, so dass das Lösungsmittel aus der Kammer 4c über den Bypass in die Kammer 4a gelangt und einen dort befindlichen Wirkstoff lösen kann. Der Stopfen 4d wird solange angetrieben, bis er auf den Stopfen 4b trifft. Dadurch wird die Abmischposition des Verabreichungsgeräts erreicht. Während diesem Vorgang wird das Aufnahmeglied 2 fortwährend weiter in das Gehäuse 1 eingeführt. Das Antriebsglied 3 bleibt jedoch in Ruhe.

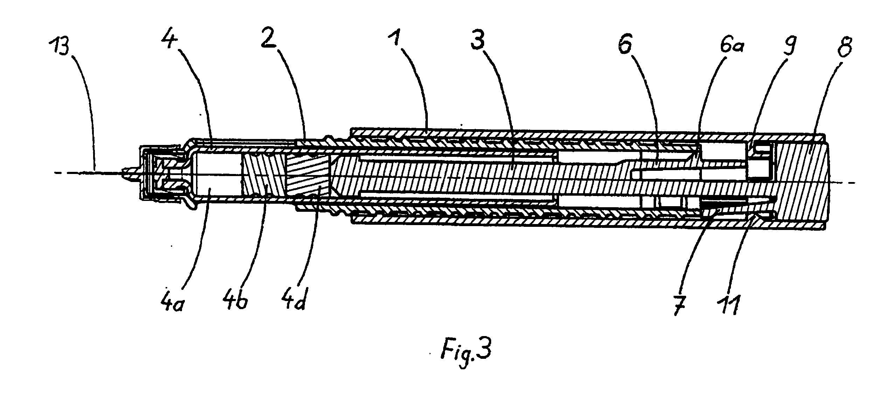

In Figur 3 ist die erfindungsgemässe Verabreichungsvorrichtung in einer Entlüftungsposition gezeigt. Aus der Abmischposition nach Figur 2 wird das Aufnahmeglied 2 weiter in proximaler Richtung in das Gehäuse 1 eingeführt. Das Aufnahmeglied 2 wurde bis hier über eine erste Einfuhrstrecke in das Gehäuse eingeführt, wobei das Halteelement 2 in der Halteposition verbleibt. Der proximale Rand des Aufnahmeglieds 2 stösst nun gegen einen Absatz 6a am Halteglied 6. Der Absatz 6a weist eine schräge Ebene auf, die gegenüber dem proximalen Ende des Aufnahmeglieds ausgerichtet ist. Wird das Aufnahmeglied 2 weiter über eine zweite Einfuhrstrecke in das Gehäuse eingeführt, stösst das proximale Ende gegen die schräge Fläche des Absatzes 6a und gleitet an dieser Fläche ab. Dabei wird das Halteelement 6 in radialer Richtung zur Achse des Antriebsglieds 3 hin ausgelenkt. Das Halteelement 6 in Form eines Funktionsarmes wird aus dem Anschlag mit dem Gehäusevorsprung 9 ausgelenkt. Von der Abmischposition in diese gelöste Position, in der das Halteelement 6 des Antriebsglieds 3 aus seiner Halteposition zum Halten des Antriebsglieds 3 relativ zum Gehäuse 1 heraus ausgelenkt wird, legt das Aufnahmeglied 2 eine zweite Einfuhrstrecke zurück, die auch als Freigabestrecke bezeichnet werden kann. Sobald das Halteelement 6 aus dem Anschlag mit dem Gehäusevorsprung 9 ausgelenkt wird, trifft das proximale Ende des Aufnahmeglieds 2 gegen das distale Ende des Aktivierungselements 7. Das Antriebsglied 3 kann nun in proximaler Richtung gegenüber dem Gehäuse 1 über eine dritte Einfuhrstrecke bewegt werden. Während einem weiteren Vorschub des Aufnahmeglieds 2 gegenüber dem Gehäuse 1 zur Auslenkung des Halteelements 6 werden gleichzeitig auch die Stopfen 4b und 4d weiter in distale Richtung des Produktreservoirs 4 verschoben. Durch die Injektionsnadel 13 kann daher überflüssige Luft aus der Produktkammer 4a entweichen. Grundsätzlich wäre es auch denkbar, den Entlüftungsvorgang beim Zurücklegen der ersten Einfuhrstrecke abzuschliessen und nur das Lösen der Halteposition des Halteelements während der zweiten und dritten Einfuhrstrecke durchzuführen.

In Figur 4A ist die erfindungsgemässe Verabreichungsvorrichtung in einer eingeführten Position gezeigt, in der das Aufnahmeglied nach Zurücklegen der dritten Einfuhrstrecke vollständig, d.h. soweit wie erforderlich, in das Gehäuse in proximaler Richtung eingeführt ist. Beim Überwinden dieser dritten Einfuhrstrecke wird das Antriebsglied 3 nicht mehr von dem Halteelement 6 gegenüber dem Gehäuse gehalten. Durch den Anschlag zwischen dem proximalen Ende des Aufnahmeglieds 2 und dem distalen Ende des Aktivierungselements 7 wird das Antriebsglied 3 über die dritte Einfuhrstrecke relativ zum Gehäuse in proximaler Richtung mitbewegt. Dabei wird der Antriebsknopf 8 aus dem proximalen Ende des Gehäuses 1 herausgefahren und ist nun für einen Benutzer zugänglich. Bei der Relativbewegung des Antriebsgliedes 3 und damit des Aktivierungselements 7 gegenüber dem Gehäuse 1 gleitet das Aktivierungselement 7 entlang der nach innen ragenden Kante des Vorsprungs 11 und wird dadurch nach innen ausgelenkt. Die dritte Einfuhrstrecke, die dem Hub des Antriebsglieds 3 aus dem Gehäuse heraus entspricht, entspricht auch der erforderlichen Vorschubstrecke beim Vorschub des Antriebsglieds in distale Richtung zur Ausschüttung des Produkts. Die dritte Einfuhrstrecke entspricht somit einem Dosierhub für die Verabreichungsvorrichtung. Entsprechend dem erforderlichen Dosierhub, kann die Einfuhrstrecke daher bei der Konstruktion der Verabreichungsvorrichtung variiert werden.

In Figur 4B ist die Detailansicht analog der Figur 1B gezeigt, in der sich die Verabreichungsvorrichtung in einer eingeführten Position befindet. Beim Anheben des Antriebsglieds 3 aus dem Gehäuse 1 wird auch der Klemmsitz zwischen dem Sicherungselement 10 und den Vorsprung 11 überwunden.

Das Aufnahmeglied 2 ist nun vollständig in das Gehäuse 1 eingeschraubt, der Wirkstoff ist abgemischt, und die Produktkammer ist entlüftet. Die Verabreichungsvorrichtung wurde in einen aktivierten Zustand mit ausgefahrenem Antriebsknopf gebracht. Sie ist nun bereit, die gewünschte Dosis des Wirkstoffes zu verabreichen.

Hierfür wird, wie in Figur 5A dargestellt, das Aufnahmeglied 2 in das Gehäuse 1 in distale Richtung eingeschoben, wobei das Aufnahmeglied 2 gegenüber dem Gehäuse 1 in Ruhe bleibt. Diese Vorschubbewegung wird daher auf die Stopfen 4b und 4d übertragen, so dass das fluide Produkt aus der Produktkammer 4a ausgeschüttet wird. Das Antriebsglied 3 wird solange in dem Gehäuse vorgeschoben, bis der Antriebsknopf 8 gegen den Gehäusevorsprung 9 anschlägt. Dabei gleitet das Aktivierungselement 7, das durch den Vorsprung 11 nach innen ausgelenkt wurde, an der Innenseite des Aufnahmeglieds 2 in das Aufnahmeglied 2 hinein. Das Halteglied 6 gleitet ebenfalls entlang der Innenfläche des Aufnahmeglieds 2. Dabei wird der Absatz 6a entlang der Umfangsfläche des Aufnahmeglieds 2 geführt. Sobald der Antriebsknopf 8 gegen den Gehäusevorsprung 9 anschlägt, ist auch das proximale Ende des Halteglieds 6 an dem Gehäusevorsprung 9 vorbei bewegt worden. In der Innenumfangsfläche des Aufnahmeglieds 2 ist in der Höhe des Absatzes 6a eine Aussparung vorgesehen, in die der Absatz 6a ausweichen kann. Durch die Vorspannung des Halteelements 6 springt dieses in die Aussparung hinein und wieder in die Halteposition, in der das distale Ende des Halteelements 6 gegen den Gehäusevorsprung 9 anstösst. Der Antriebsknopf 8 ist wieder innerhalb des Gehäuses 1 versenkt.

Die Verabreichungsvorrichtung befindet sich nun in einer Endposition. Das Halteelement 6 ist dabei wieder in einer Halteposition, sodass das Antriebsglied 3 gegenüber dem Gehäuse 1 festgehalten wird. Die Verabreichungsvorrichtung ist daher gegen jegliche weitere Betätigung blockiert. In Figur 5B ist das Detail aus Figur 1B mit der Verabreichungsvorrichtung in einer Endposition gezeigt. Es ist ersichtlich, dass das Sicherungselement 10 wieder einen Klemmsitz mit dem Vorsprung 11 des Gehäuses 1 eingeht Dadurch wird zusätzlich zu dem Haltegriff zwischen Halteelement 6 und Gehäusevorsprung 9 das Antriebsglied 3 gegen weitere Bewegungen oder ein Wackeln gesichert Die Verabreichungsvorrichtung kann nun in diesem gesicherten Zustand entsorgt werden.

Es wird ausdrücklich darauf hingewiesen, dass es für den Zweck der vorliegenden Erfindung ausreicht, das Antriebsglied 3 durch das Halteelement 6 gegen ein proximales Bewegen relativ zum Gehäuse zu sichern. Das Sicherungselement 10 dient lediglich einer komfortableren Bedienung und einer zusätzlichen Führung für das Antriebsglied 3.

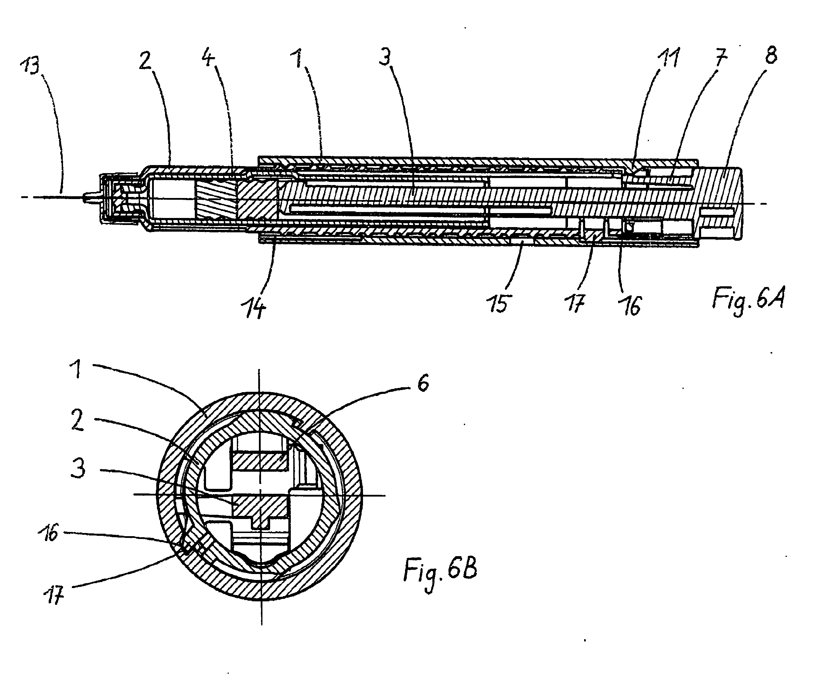

In Figur 6A ist ein Schnitt durch eine erfindungsgemässe Verabreichungsvorrichtung gezeigt, aus welcher die Rasteinrichtung, bzw. Anzeigenvorrichtung zur Verrastung und Anzeige bei den einzelnen Vorbereitungsschritten der Verabreichungsvorrichtung gezeigt ist. In Figur 6A ist die Verabreichungsvorrichtung bereits in einer Abmischposition. Die Anzeigen- oder Rasteinrichtung umfasst eine erste Rastrille 14, eine zweite Rastrille 15, die auch als Rastöffnung ausgebildet sein kann, und eine dritte Rastrille 16. Am Aufnahmeglied 2 ist ein in Umfangsrichtung ausgerichteter Rastarm 17 vorgesehen, der derart radial nach aussen vorgespannt ist, dass er beim Eindrehen des Aufnahmeglieds 2 in das Gehäuse 1 entlang der Innenumfangsfläche des Gehäuses gleitet. Beim Einsetzen des Aufnahmegliedes 2 in das Gehäuse 1 wird das Aufnahmeglied 2 soweit in das Gehäuse 1 eingedreht, bis der Rastarm 17 mit der ersten Rastrille 14 verrastet. In dieser Verrastung befindet sich die Verabreichungsvorrichtung in der Anfangsposition. Das Verrasten erzeugt vorteilhafterweise ein akustisches Signal, welches das Erreichen der Anfangsposition anzeigt. Der Rastarm 17 ist derart ausgebildet, dass er auf Grund seiner Vorspannung ein Wiederherausdrehen, bzw. ein Zurückdrehen, des Aufhahmeglieds 2 aus dem Gehäuse heraus durch Anschlag an die erste Rastrille 14 blockiert. Aus der Anfangsposition ist es daher nur möglich das Aufnahmeglied 2 weiter in proximale Richtung in das Gehäuse 1 einzudrehen. Grundsätzlich ist es möglich, die erste Rastrille derart über die Länge des Gehäuses anzuordnen, dass nach jeder Umdrehung des Aufnahmeglieds eine Verrastung und vorteilhaft auch ein damit verbundenes akustisches Signal eintritt. Aus der Anfangsposition wird das Aufnahmeglied weiter eingeführt bis es in der zweiten Rastöffnung 15 mit dem Rastarm 17 verrastet, wodurch die Beendigung des Abmischens des Zweikammerreservoirs angezeigt wird. Ist diese zweite Rastrille als Öffnung ausgebildet, ist es grundsätzlich möglich, dass der Rastarm 17 von aussen aus der Rastöffnung ins Innere eingedrückt wird, wodurch das Aufnahmeglied auch wieder aus dem Gehäuse 1 herausgedreht werden könnte. Diese Möglichkeit erscheint grundsätzlich sinnvoll, wenn nach dem Abmischen nicht unmittelbar eine Verabreichung aus der Verabreichungsvorrichtung erfolgen soll. Im Allgemeinen, wird diese zweite Rastrille jedoch nicht als Öffnung ausgebildet, so dass auch diese zweite Verrastung eine Rückdrehsicherung für das Aufnahmeglied aus dem Gehäuse darstellt. Aus dieser Abmischposition kann das Aufnahmeglied weiter in proximaler Richtung in das Gehäuse eingeschraubt werden, bis der Rastarm 17 in der driftten Rastrille 16 verrastet ist, wodurch die Entlüftungsposition angezeigt wird. Die Verrastung zwischen dem Rastarm 17 und der dritten Rastrille 16 bildet gleichfalls eine Rückdrehsicherung, so dass das Aufnahmeglied in dieser Position gegen jegliche weitere Bewegung gegenüber dem Gehäuse blockiert ist, da die Aufnahmehülse 2 zugleich gegen den Gehäusevorsprung 9 in axialer Richtung anschlägt.

In Figur 6B ist ein Querschnitt durch die Verabreichungsvorrichtung gezeigt, in welcher der Rastarm 17 das Aufnahmeglied 2 in einer Rastposition mit einer Rastrille im Gehäuse 1 gezeigt ist. Aus Figur 6B ist ersichtlich, dass ein Verdrehen des Aufnahmegliedes 2 entgegen dem Uhrzeigersinn durch die Rastung verhindert wird, wohingegen ein Drehen des Aufnahmeglieds 2 innerhalb des Gehäuses 1 im Uhrzeigersinn ermöglicht wird, in dem der Rastarm 17 flexibel nach innen ausweicht und entlang der Schräge der Rille aus der Rille herausgleiten kann.

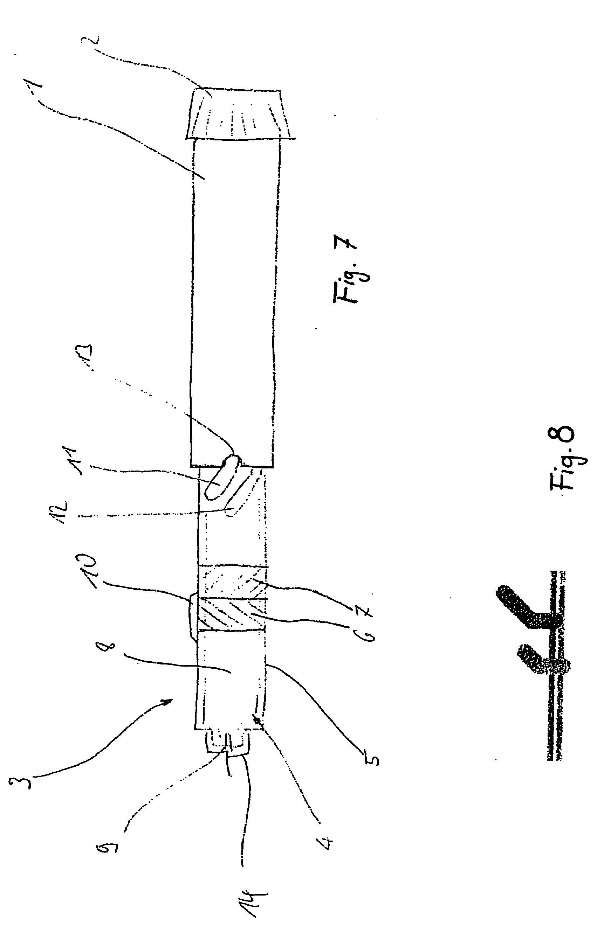

In Figur 7 ist eine zweite Ausführungsform einer erfindungsgemässen Verabreichungsvorrichtung mit einem Gehäuse 1, einem Antriebsknopf 8 zur Durchführung der Verabreichung, und einem Aufnahmeglied 2 mit einer Zweikammerkarpule 4 gezeigt. Die Zweikammerkarpule weist zwei Stopfen 4b und 4d auf. In der in der Darstellung links gezeigten ersten Kammer 4a der Karpule zwischen der Wand der Karpule, einer vorderen Öffnung in der Karpule und dem Stopfen 4d befindet sich der Wirkstoff. In Figur 7 ist die Zweikammerkarpule bereits in einer abgemischten Position gezeigt, so dass sich zwischen dem Stopfen 4b und dem Stopfen 4d keine Kammer mit einer Lösungsflüssigkeit mehr befindet, da die Lösungsflüssigkeit durch einen Bypass 12 in der Karpulenwand aus der zweiten Kammer in die erste Kammer 4a befördert wurde.

Auf dem Aufnahmeglied 2 ist eine erste Anzeige 21 in Form eines Fähnchens aufgebracht, welche die Stellung der Karpule 4 relativ zum Gehäuse 1 nach der Beendigung des Abmischens angibt. Neben dem Fähnchen 11 ist eine zweite Anzeige 22 in Form eines weiteren Fähnchens dargestellt, das der Markierung der Stellung der Karpule relativ zum Gehäuse nach einer Entlüftung dient.

In Figur 8 sind die beiden Fähnchen 21 und 32 schematisch dargestellt. Die Fähnchen können beispielsweise durch einen Aufkleber auf der Karpulenhülse oder auf der Karpule selbst angeordnet werden. Die Fähnchen unterscheiden sich in ihrer Farbe und haben entsprechend ihrer Funktion eine unterschiedliche Beschriftung. Sie befinden sich entlang der Längsachse des Verabreichungsgeräts und in Umfangsrichtung des Verabreichungsgeräts zueinander versetzt.

In Figur 7 ist am linken Rand des Gehäuses 1 der Verabreichungsvorrichtung eine Aussparung 23 vorgesehen, welche als Zeiger zum Anwählen bzw. identifizieren der ersten und zweiten Anzeige, bzw. der Fähnchen, 21 und 22, dient. Der Zeiger in Form der Aussparung 23 ist demnach gehäusefest, wobei die Anzeigen 21 und 22 in Form der Fähnchen auf der Karpulenhülse relativ zu dem Gehäuse beweglich sind. Das in Figur 7 gezeigte Gerät ist bereits abgemischt, aber noch nicht geprimt, bzw. entlüftet.

Wird nun das Aufnahmeglied 2 zum Abmischen der Zweikammerkarpule 4 in das Gehäuse 1 eingedreht, wandern die Fähnchen 21 und 22 durch die Drehbewegung sowohl axial als auch in Umfangsrichtung relativ zum Gehäuse. Nach Beendigung des Abmischens der Zweikammerkarpule kommt das Fähnchen 21 innerhalb der Aussparung 23 zu liegen. Ein Anwender kann daher in einfacher Weise ablesen, dass der Abmischvorgang vollständig ausgeführt wurde. Durch weiteres Eindrehen des Aufnahmeglieds 2 in das Gehäuse 1 werden die Stopfen 4b und 4d weiter innerhalb der Zweikammerkarpule 4 in Richtung auf die Öffnung vorgeschoben und eine in der ersten Kammer 4a der Karpule verbliebene Luftrestmenge kann durch die Öffnung und die aufgesetzte Nadeleinheit 13 entweichen. Nach Beendigung der Drehung für die Entlüftung kommt die zweite Anzeige mit dem Fähnchen 22 in der Aussparung 23 zu liegen. Es ist somit für den Anwender ersichtlich, dass auch der Entlüftungsvorgang vollständig abgeschlossen ist. Die Verabreichungsvorrichtung mit der Zweikammerkarpule ist nun zur Verabreichung des abgemischten Wirkstoffes bereit.

### Bezugszeichen:

- 1: Gehäuse
- 2: Aufnahmeglied
- 3: Antriebsglied
- 4: Produktreservoir
- 4a: Erste Kammer
- 4b: Erster Stopfen
- 4c: Zweite Kammer
- 4d: Zweiter Stopfen
- 5a: Führungsnut
- 5b: Führungsnocken
- 6: Halteelement
- 6a: Absatz
- 7: Aktivierungselement
- 8: Antriebsknopf
- 9: Gehäusevorsprung
- 10: Sicherungselement
- 11: Vorsprung
- 12: Bypass
- 13: Injektionsnadel
- 14: erste Rastrille
- 15: zweite Rastöffnung
- 16: dritte Rastrille
- 17: Rastarm

- 21: erste Anzeige
- 22: zweite Anzeige
- 23: Aussparung

## Patentansprüche

1. Vorrichtung zur Verabreichung eines Wirkstoffes aus einer Mehrkammerkarpule (4) mit wenigstens einer ersten Kammer (4a) mit einem festen Wirkstoff und einer zweiten Kammer (4c) mit einer Lösungsflüssigkeit für den Wirkstoff, die eine Mischvorrichtung zum Mischen des Wirkstoffes und der Lösungsflüssigkeit und ein Gehäuse (1) zur Aufnahme der Mischvorrichtung aufweist,
**dadurch gekennzeichnet, dass** eine Anzeigenvorrichtung vorgesehen ist, die zumindest eine visuell wahrnehmbare Markierung als erste Anzeige (15; 21) zur Markierung oder Anzeige einer Position der Mehrkammerkarpule (4) relativ zum Gehäuse (1), welche nach Beendigung des Abmischens erscheint und eine visuell wahrnehmbare Markierung als zweite Anzeige (16; 22) zur Markierung oder Anzeige einer Position der Karpule (4) relativ zum Gehäuse (1), welche nach Beendigung eines Entlüftungsvorgangs der Karpule (4) erscheint, umfasst.

2. Verabreichungsvorrichtung nach Anspruch 1, wobei die Anzeigenvorrichtung eine dritte Anzeige (14) zur Markierung einer Anfangsposition der Karpule (4) relativ zum Gehäuse (1) vor einem Abmischvorgang umfasst.

3. Verabreichungsvorrichtung nach Anspruch 1 oder 2, wobei die Anzeigen (14; 15,21; 16; 22) axial versetzt entlang einer Längsachse der Verabreichungsvorrichtung und/oder versetzt in Umfangsrichtung auf dem Umfang der Verabreichungsvorrichtung angeordnet sind.

4. Verabreichungsvorrichtung nach einem der vorhergehenden Ansprüche, wobei ein Aufnahmeglied (2) vorgesehen ist, der zum Abmischen des Wirkstoffes und der Lösungsflüssigkeit relativ zum Gehäuse (1) beweglich ist und die Karpule (4) in sich aufnimmt.

5. Verabreichungsvorrichtung nach einem der vorhergehenden Ansprüche, wobei die Anzeigen der Anzeigevorrichtung durch eine Kombination aus visueller Markierung und akustischem Signal gegeben sind.

6. Verabreichungsvorrichtung nach einem der vorhergehenden Ansprüche, wobei an einem aus Aufnahmeglied (2) und Gehäuse (1) ein Zeiger oder eine Aussparung angeordnet ist, wodurch eine der Anzeigen (15, 21; 16; 22) anzeigbar ist, und der andere aus Aufnahmeglied (2) und Gehäuse (1) die Anzeigenvorrichtung aufweist.

7. Verabreichungsvorrichtung nach einem der vorhergehenden Ansprüche, wobei die Anzeigenvorrichtung durch eine Rasteinrichtung (14, 15, 16; 17) gegeben ist.

8. Verabreichungsvorrichtung nach Anspruch 7, wobei die Rasteinrichtung eine lösbare oder unlösbare Raststellung zwischen dem Aufnahmeglied (2) und dem Gehäuse (1) derart bildet, dass eine Bewegung entgegen einer Einführbewegung des Aufnahmeglieds (2) in das Gehäuse (1) blockiert ist.

9. Verabreichungsvorrichtung nach einem der Ansprüche 7 oder 8, wobei die Einführbewegung zwischen Aufnahmeglied (2) und Gehäuse (1) eine Drehbewegung ist und die Rasteinrichtung eine Rückdrehsicherung bildet.

10. Verfahren zur Bereitstellung einer Verabreichungsvorrichtung mit einer Mehrkammerkarpule (4), wobei die Beendigung eines Abmischvorgangs der Mehrkammerkarpule (4) durch eine visuell wahrnehmbare Markierung als erste Anzeige (11) angegeben wird, welche nach Beendigung des Abmischens erscheint, und die Beendigung eines Entlüftungsvorgangs aus der Mehrkammerkarpule (4) durch eine visuell wahrnehmbare Markierung als zweite Anzeige (16;22) angegeben wird, welche nach Beendigung des Entlüftungsvorgangs der Karpule (4) erscheint.

11. Verfahren nach Anspruch 10, wobei die Ausgangsstellung der Mehrkammerkarpule (4) vor dem Beginn des Abmischvorgangs durch eine dritte Anzeige (14) angegeben wird.

## Claims

1. Device for administering an active substance from a multi-chamber carpule (4) with at least one first chamber (4a) holding a solid active substance and a second chamber (4c) holding a dissolving liquid for the active substance, which has a mixing device for mixing the active substance with the dissolving liquid and a housing (1) accommodating the mixing device,
**characterised in that** a display device is provided which comprises at least one visually perceivable marking arrangement as a first display (15; 21) for marking or indicating a position of the multi-chamber carpule (4) relative to the housing (1) which display appears after the end of the mixing and a visually perceivable marking arrangement appears as a second display (16; 22) for marking or indicating a position of the carpule (4) relative to the housing (1), which display appears after a process removing air from the carpule (4).

2. Administering device according to claim 1 wherein the display device comprises a third display (14) for marking a starting position of the carpule (4) relative to the housing (1) before a mixing process.

3. Administering device according to claim 1 or 2 wherein the displays (14; 15, 21; 16; 22) are arranged to be axially offset along a longitudinal axis of the administering device and/or offset in a circumferential direction on the circumference of the administering device.

4. Administering device according to any one of the preceding claims wherein a holder element (2) is provided which is movable relative to the housing (1) for the mixing of the active substance and the dissolving liquid and which accommodates the carpule (4).

5. Administering device according to any one of the preceding claims wherein the displays of the display device are provided by a combination of visual marking and an acoustic signal.

6. Administering device according to any one of the preceding claims wherein a pointer or recess is arranged associated with holder element (2) and housing (1) by which one of the displays (15, 21; 16; 22) can be displayed, and the other associated with the holder element (2) and housing (1) having the display device.

7. Administering device according to any one of the preceding claims wherein the display device is provided by a latch device (14, 15,16; 17).

8. Administering device according to claim 7 wherein die latch device forms a releasable or lockable latch position between the holder element (2) and the housing (1) such that a movement relative to an insertion movement of the holder element (2) into the housing (1) is blocked.

9. Administering device according to one of the claims 7 or 8 wherein the insertion movement between holder element (2) and housing (1) is a rotational movement and the latch device acts to prevent rotation in the reverse direction.

10. Method for producing an administering device with a multi-chamber carpule (4) wherein the end of the mixing process by the multi-chamber carpule (4) is indicated by a visually perceivable marking arrangement as a first display (11) which appears after the end of the mixing process, and the end of the removal of air from the multi-chamber carpule (4) is indicated by a visually perceivable marking arrangement as a second display (16;22) which appears after the end of the process to remove air from the carpule (4).

11. Method according to claim 10 wherein the initial position of the multi-chamber carpule (4) is indicated by a third display (14) before the start of the mixing process.

## Revendications

1. Dispositif servant à administrer un principe actif depuis une carpule à cavités multiples (4) comprenant au moins une première cavité (4a) présentant un principe actif solide et une deuxième cavité (4c) présentant un liquide de dissolution pour le principe actif, lequel dispositif présente un dispositif de mélange servant à mélanger le principe actif et le liquide de dissolution et un boîtier (1) servant à recevoir le dispositif de mélange, **caractérisé en ce qu'**est prévu un dispositif indicateur, qui comprend au moins un marquage visuellement perceptible faisant office de première indication (15 ; 21) servant à marquer ou à indiquer une position de la carpule à cavités multiples (4) par rapport au boîtier (1), lequel marquage apparaît après la fin du mélange, et un marquage visuellement perceptible faisant office de deuxième indication (16 ; 22) servant à marquer ou à indiquer une position de la carpule (4) par rapport au boîtier (1), lequel marquage apparaît après la fin d'une opération d'évacuation de l'air de la carpule (4).

2. Dispositif d'administration selon la revendication 1, le dispositif indicateur comprenant une troisième indication (14) servant à marquer une position de départ de la carpule (4) par rapport au boîtier (1) avant une opération de mélange.

3. Dispositif d'administration selon la revendication 1 ou 2, les indications (14 ; 15, 21 ; 16 ; 22) étant disposées de manière axialement décalée le long d'un axe longitudinal du dispositif d'administration et/ou de manière décalée dans la direction périphérique sur la périphérie du dispositif d'administration.

4. Dispositif d'administration selon l'une quelconque des revendications précédentes un organe de réception (2) étant prévu, lequel peut être déplacé par rapport au boîtier (1) afin de mélanger le principe actif et le liquide de dissolution et qui accueille la carpule (4).

5. Dispositif d'administration selon l'une quelconque des revendications précédentes, les indications du dispositif indicateur étant données par une combinaison composée d'un marquage visuel et d'un signal acoustique.

6. Dispositif d'administration selon l'une quelconque des revendications précédentes, une aiguille ou un évidement étant disposé au niveau de l'un des éléments parmi l'organe de réception (2) et le boîtier (1), ce qui permet d'indiquer une des indications (15, 21 ; 16 ; 22), et l'autre élément parmi l'organe de réception (2) et le boîtier (1) présentant le dispositif indicateur.

7. Dispositif d'administration selon l'une quelconque des revendications précédentes, le dispositif indicateur étant donné par un système d'arrêt (14, 15, 16 ; 17).

8. Dispositif d'administration selon la revendication 7, le système d'arrêt formant une position d'arrêt amovible et non amovible entre l'organe de réception (2) et le boîtier (1) de telle manière qu'un déplacement en sens inverse d'un déplacement d'introduction de l'organe de réception (2) dans le boîtier (1) est bloqué.

9. Dispositif d'administration selon l'une quelconque des revendications 7 ou 8, le déplacement d'introduction entre l'organe de réception (2) et le boîtier (1) étant un déplacement par rotation et le système d'arrêt formant un système de sécurité anti-rotation en sens inverse.

10. Procédé servant à fournir un dispositif d'administration comprenant une carpule à cavités multiples (4), la fin d'une opération de mélange de la carpule à cavités multiples (4) étant indiquée par un marquage visuellement perceptible faisant office de première indication (11), lequel marquage apparaît à la fin du mélange, et la fin d'une opération d'évacuation de l'air de la carpule à cavités multiples (4) étant indiquée par un marquage visuellement perceptible faisant office de deuxième indication (16 ; 22), lequel marquage apparaît après la fin de l'opération d'évacuation de l'air de la carpule (4).

11. Procédé selon la revendication 10, la position de départ de la carpule à cavités multiples (4) étant indiquée par une troisième indication (14) avant le début de l'opération de mélange.
